# EUROPÄISCHE PATENTSCHRIFT

(11) **EP 3 232 808 B1**
(45) Veröffentlichungstag und Bekanntmachung des Hinweises auf die Patenterteilung: **01.04.2020**
(21) Anmeldenummer: 15825932.5
(22) Anmeldetag: 21.12.2015
(51) Int. Cl.: A23L 33/105, A23L 2/52, A23L 35/00, A61K 31/352, A61K 36/185, A61K 31/05

(54) **CBD-HALTIGES GETRÄNK**
CBD CONTAINING BEVERAGE
BOISSON CONTENANT DU CBD

(30) Priorität: 19.12.2014 EP 14199135
(43) Veröffentlichungstag der Anmeldung: 25.10.2017
(73) Patentinhaber: THC Pharm GmbH The Health Concept, 60599 Frankfurt am Main (DE)
(72) Erfinder: STEUP, Christian, 65719 Hofheim (DE)
(74) Vertreter: Mewburn Ellis LLP
(86) Internationale Anmeldenummer: PCT/EP2015/080894
(87) Internationale Veröffentlichungsnummer: WO 2016/097425

(56) Entgegenhaltungen:
- EP-A1- 2 444 081
- GB-A- 2 377 633
- US-A1- 2007 049 645
- US-A1- 2009 047 234
- US-A1- 2013 184 354
- DATABASE GNPD [Online] MINTEL; 1. April 2014 (2014-04-01), "Cannabis Moodlifter Drink", XP002756858, Database accession no. 2369092
- DATABASE GNPD [Online] MINTEL; 1. Januar 2004 (2004-01-01), "The Hempy Cola", XP002756859, Database accession no. 250086
- CARY LEIZER ET AL: "The composition of Hemp Seed Oil and Its potential as an Important Source of Nutrition", JOURNAL OF NUTRACEUTICALS, FUNCTIONAL AND MEDICAL FOODS, PHARMACEUTICAL PRODUCTS PRESS, BINGHAMTON, NY, US, Bd. 2, Nr. 4, 1. Januar 2000 (2000-01-01), Seiten 35-53, XP002736420, ISSN: 1089-4179
- ANTONIO WALDO ZUARDI: "Cannabidiol: from an inactive cannabinoid to a drug with wide spectrum of action", REVISTA BRASILEIRA DE PSIQUIATRIA, Bd. 30, Nr. 3, 1. September 2008 (2008-09-01), Seiten 271-280, XP055267158, BR ISSN: 1516-4446

## Beschreibung

Die Erfindung betrifft ein Getränk oder dessen Grundstoff enthaltend eine Flüssigformulierung umfassend Cannabidiol und Wasser und mindestens einen Emulgator, wobei das Cannabidiol synthetisches oder aufgereinigtes Cannabidiol aus einem Cannabis-Extrakt ist, aufweisend 50mg/L bis 300 mg/L CBD.

Cannabidiol (kurz: CBD) ((2-[1R-3-Methyl-6-(1-methylethenyl]-2-cyclohexen-1-yl]-5-pentyl-1,3-benzoldiol)) ist ein schwach psychoaktives Cannabinoid aus dem weiblichen Hanf Cannabis sativa / indica. CBD wirkt entkrampfend, entzündungshemmend, angstlösend und gegen Übelkeit (Übersicht in: Zuardi AW, Cannabidiol: from an inactive cannabinoid to a drug with wide spectrum of action, Rev Bras Psiquiatr. 2008 Sep;30(3):271-80) . 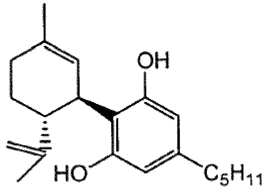 CBD-Strukturformel

Im Stand der Technik sind verschiedene Darreichungsformen und Formulierungen für CBD beschrieben und deren Applizierung, wie z.B. EP1289517B1, WO2008019146, welche jedoch allesamt eine zumindest teilweise feste Formulierung betreffen. Ferner ist bekannt, dass CBD in wässriger Lösung einen starken Bittergeschmack verursacht. Hanfextrakt wird bekannter Weise in Getränken verwendet (z.B. "Cannabis Moodlifter Drink", GNPD database, Mintel Ltd.No. 2369092, https://www.gnpd.com/sinatra/recordpage/2369092/from_search/YwkWuL145E/?page=1) Aufgabe der vorliegenden Formulierung ist die Bereitstellung einer Flüssig-Formulierung, die als Getränk einem Menschen oder Tier verabreicht werden kann.

Die Aufgabe wird gelöst durch den Patentanspruch 1.

Eine weitere Aufgabe betrifft die Bereitstellung von CBDhaltigen Flüssig-Formulierungen mit einer deutlich erhöhten Menge an CBD, die dennoch wohlschmeckend sind und nicht den bitteren CBD-Geschmack aufweisen und oral verabreicht werden können.

Gegenstand der Erfindung sind daher CBD-haltige Getränke, Grundstoffe als auch Verfahren zur Herstellung und deren Verwendung.

Überaschenderweise wird durch die Kombination von CBD und mindestens einem Emulgator ein Getränk erhalten, in dem CBD in sehr hohen Konzentrationen stabil gelöst ist, und auch bei langer Lagerung oder Vorhaltung nicht ausfällt.

Der Emulgator ist besonders bevorzugt ausgewählt aus der Gruppe bestehend aus Polysorbaten, wie beispielsweise kommerziell erhältlichen Polyoxyethylen-Sorbitanfettsäurester (Tween)- und Span-Produkten, insbesondere Tween 20, 60, 80, Gummen wie beispielsweise Gummi arabicum, Pektinen, Stärken und modifizierten Stärken wie beispielsweise Purity Gum und/oder Proteinen wie beispielsweise Caseinaten aus Milch.

In einer bevorzugten Ausführungsform ist der Emulgator ein gereinigter Emulgator oder eine Mischung aus Emulgatoren, die im Wesentlichen nicht weitere Substanzen enthält. Gemäß dieser bevorzugten Ausführungsform ist der Emulgator nicht Bestandteil einer Mischung von Stoffen, die Emulgatoren beinhaltet, wie zum Beispiel Milchpulver oder Trockenmilch. Emulgatoren können vorteilhaft weiterhin ausgewählt werden aus der Gruppe der nichtionischen, anionischen, kationischen oder amphoteren Emulgatoren.

Als nichtionische Emulgatoren können verschiedene Emulgatoren aus den Gruppen der Partialfettsäureester, Fettalkohole, Sterole, Polyethylengylcole wie z.B. ethoxylierte Fettsäuren, ethoxylierte Fettalkohole und ethoxylierte Sorbitanester, Zuckeremulgatoren, Polyglycerinemulgatoren oder Silikonemulgatoren Verwendung finden.

Als anionische Emulgatoren können verschiedene Emulgatoren aus den Gruppen der Seifen z. B. Natriumstearat, Fettalkoholsulfate, Mono-, Di- und Trialkylphosphosäureester und deren Ethoxylate, Fettsäure Lactat Ester, Fettsäure Citrat Ester oder Fettsäure Citroglycerinester Verwendung finden.

Als kationische Emulgatoren können beispielsweise quaternäre Ammoniumverbindungen mit einem langkettigen aliphatischen Rest z. B. Distearyldimonium Chloride eingesetzt werden.

Unter den amphoteren Emulgatoren können verschiedene Emulgatoren aus den Gruppen Alkylamininoalkancarbonsäuren, Betaine, Sulfobetaine oder Imidazolinderivate Verwendung finden.

Erfindungsgemäß bevorzugt sind weiterhin natürlich vorkommende Emulgatoren, zu denen beispielsweise Bienenwachs, Lecithin und Sterole u.a. gehören, die ebenfalls bei der Herstellung einer erfindungsgemäßen Flüssig-Formulierung eingesetzt werden können.

Daher betrifft die Erfindung eine Flüssig-Formulierung enthaltend Cannabidiol und Wasser und mindestens einen Emulgator.

Erfindungsgemäß wird das CBD-haltige Getränk vorzugsweise hergestellt, indem das CBD in Form einer stabilen Emulsion in das Fertiggetränk oder den entsprechenden Getränkegrundstoff eingebracht wird. Das fertige Getränk muss keine Emulsion sein, obwohl solche erfindungsgemäßen Ausführungsformen ebenfalls umfasst sind. Emulgatoren und Wasser können in beliebiger Menge zum Cannabidiol zugesetzt werden.

In einer bevorzugten Ausführungsform wird das CBD vorzugsweise synthetisch hergestellt, entsprechend der Lehre der Anmelderin aus EP 2314580 A1. Ebenfalls kann das CBD aus Cannabis-Extrakten gewonnen und nach Aufreinigung eingesetzt werden (Siehe z.B. DE10051427C1).

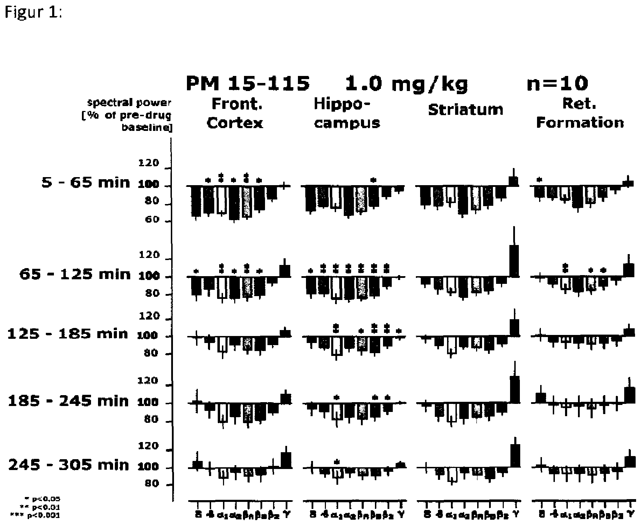

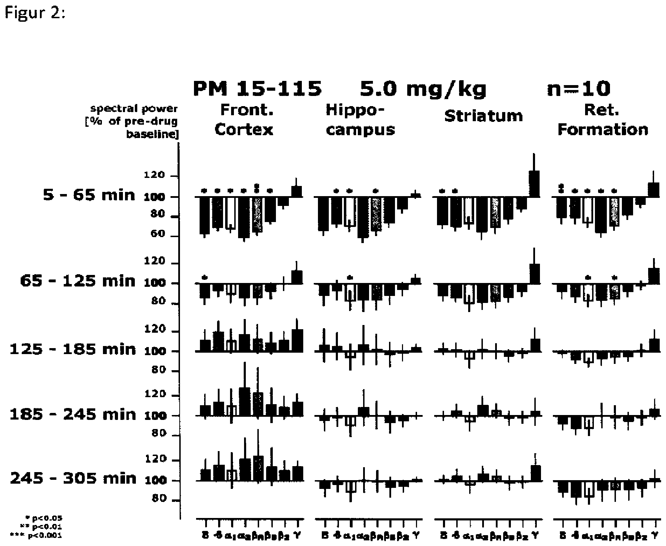

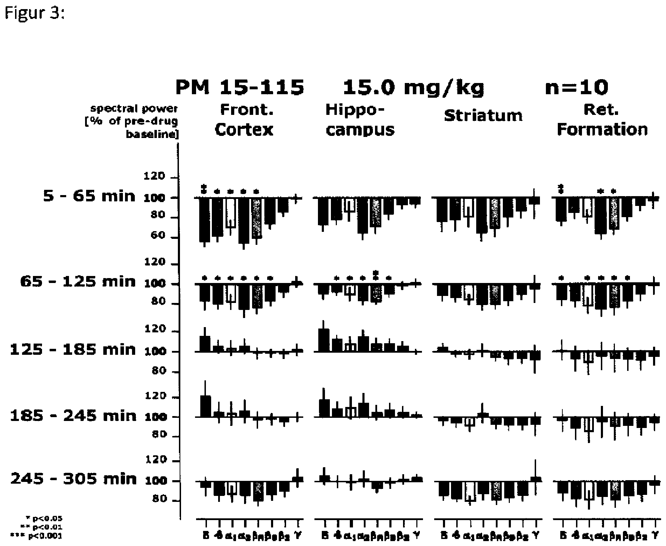

Die Figuren 1 bis 3 zeigen Electropharmakogramme aus EEG (Elektroenzephalo-gramm/-grafie) von erwachsenen Ratten (> 3 Monate alt), wobei eine Dosis von 1 mg/kg (Körpergewicht) (Figur 1), 5 mg/kg (Körpergewicht) (Figur 2), 15 mg/kg (Körpergewicht) (Figur 3) Cannabidiol in einer flüssigen Formulierung verabreicht wurde.

Hierbei wurden unter Vollnarkose vier Tiefenelektroden in den frontalen Cortex, den Hippocampus, das Striatum sowie in die Formatio Reticularis der Ratten implantiert. Vom Kopf der Ratte werden die Signale drahtlos per Funk an einen Computer übertragen. Gleichzeitig wird die Bewegungsaktiviät gemessen.

Die Signale werden einer Frequenzanalyse unterworfen z.B. unter Verwendung der Fast Fourier Transformation (FFT), und der Frequenzgehalt eines Signals wird bestimmt. Bei EEG Daten wird das Spektrum in mehrere Bereiche unterteilt, die historisch mit delta, theta, alpha und beta bezeichnet werden.

Nach Unterteilung in sieben Frequenzbereiche ((1) bis (7), siehe Figuren 1-3) kann man die Änderungen der elektrischen Leistung nach Gabe von einer erfindungsgemäßen flüssigen Formulierung enthaltend Cannabidiol quantifizieren und erhält unter Berücksichtigung der Signale der vier Hirnregionen ein spezifisches Veränderungsmuster, das "Elektropharmakogramm (supra)" genannt wird (Dimpfel W., Pharmacological classification of herbal extracts by means of comparison to spectral EEG signatures induced by synthetic drugs in the freely moving rat. Journal of Ethnopharmacology 2013, 149, 583-589).

Ein Anstieg von gamma spectral Power (7) (siehe Figur 1) kann bei einer Dosis von 1 mg/kg bis 5 mg/kg innerhalb des Striatum, weniger im Formatio reticularis und frontalen Cortex, und nicht im Hippocampus festgestellt werden. Dieser spezifische Anstieg ist zumindest für 4 Stunden sichtbar. Gamma spectral Power korreliert mit gesteigerter Bewegung (Dimpfel W (2015) Drug Discovery and Translational Medicine. Neurophysiological Techniques Provide a Holistic Approach to Saving Animals. BoD Verlag Norderstetten). Hingegen wird bei einer Dosis von 15 mg/kg CBD (Figur 3) kein spezifischer Anstieg von gamma spectral Power festgestellt.

Im Ergebnis haben hohe Dosierungen von Cannabidiol in einer flüssigen Formulierung und zwar 5 oder 15 mg/kg (Körpergewicht) gemäß Figur 2 und 3 einen sedierenden Charakter. Das EEG Muster bzw. Electropharmakogramm entspricht dem bestimmter Antidepressiva und Analgetika. Dies wird bestätigt durch die signifikante Abnahme des Bewegungsindex. Bei niedrigster Dosierung, insbesondere kleiner als 5 mg/kg (Körpergewicht), kleiner als 2 mg/kg (Körpergewicht), vorzugsweise 1 mg/kg (Körpergewicht) wurde eine aktivierende und belebende Wirkung festgestellt.

Eine flüssig-Formulierung enthaltend Cannabidiol und Wasser und mindestens einen Emulgator, entsprechend der Getränkezusammensetzung der vorliegenden Erfindung kann einem Menschen oder Tier verabreicht/appliziert werden, wobei eine Dosis von 1 mg/kg bis 5 mg/kg (Körpergewicht) CBD verwendet wird. In einer bevorzugten Ausführungsform können daher in einer flüssigen Formulierung insbesondere 50 mg/L bis 100 mg/L CBD in einer erfindungsgemäßen Formulierung vorliegen. Beispielsweise erlaubt die Verabreichung von 60 mg/L CBD einer flüssigen Formulierung eine Dosierung von 1 mg/kg (Körpergewicht) bei 60 kg Körpergewicht.

Als positive Eigenschaften entfaltet ein entsprechendes Getränk einen leistungsfördernden, belebenden, bekömmlichen, wohltuenden, jedoch nicht-psychoaktiven Effekt für Mensch oder Tier. Diese Effekte können sich bereits ab 10 mg/L CBD in einer flüssigen Formulierung einstellen.

Weitere positive Wirkungen sind eine Steigerung der Konzentrationsfähigkeit, Steigerung der geistigen Leistungsfähigkeit, Steigerung der Erinnerungsleistung.

Daher betrifft die Erfindung ein Nahrungsergänzungsmittel umfassend die flüssig-Formulierung enthaltend Cannabidiol und Wasser und mindestens einen Emulgator entsprechend der Getränkezusammensetzung der vorliegenden Erfindung zur Steigerung der Konzentrationsfähigkeit, Steigerung der körperlichen, sportlichen und geistigen Leistungsfähigkeit und Steigerung der Erinnerungsleistung, ggfs. samt Hilfs- und Zusatzstoffen.

Daher betrifft die Erfindung einen Energy-Drink aufweisend eine flüssig-Formulierung enthaltend Cannabidiol und Wasser und mindestens einen Emulgator samt Hilfs- und Zusatzstoffen, gemäß der Getränkezusammensetzung der vorliegenden Erfindung.

Aufgrund der unerwarteten positiven Eigenschaften berücksichtigt die vorliegende Erfindung als weitere Ausführungsform die Möglichkeit, dass die Formulierung oder das Getränk als Mineralwasser, Limonade, Tonicwater, Sport-, Mineral-, Frucht-, Fruchtsaft-, Milch-, Molke, Soft- oder alkoholhaltiges Getränk, wie Bier oder als Trinkwasserzubereitung vorliegt. Im weitesten Sinne betrifft die vorliegende Erfindung ein Life-Style-Produkt, insbesondere ein Produkt zur Leistungsförderung.

Darüber hinaus betrifft die Erfindung ebenfalls ein Aromamittel enthaltend Cannabidiol in einer Flüssig-Formulierung gemäß der Getränkezusammensetzung der vorliegenden Erfindung. Ein solches erfindungsgemäßes Aromamittel kann beispielsweise als Chinin-Ersatz verwendet werden. Daher betrifft die Erfindung ein Aromamittel umfassend eine flüssig-Formulierung enthaltend Cannabidiol und Wasser und mindestens einen Emulgator.

In einer weiteren Ausführungsform kann die flüssige Formulierung oder das Getränk mit Aromen versetzt werden. Die Aromen dienen vorzugsweise zur Maskierung des Bittergeschmackes und der Fachmann ist in der Lage entsprechende Aromen bereitzustellen. Beispiele für Aromen sind nicht-abschließend vorzugsweise Lebensmittelaromen, wie z.B. in VO (EG) Nr. 1334/2008 beschrieben. Insbesondere sind Fruchtaromen bevorzugt, wie Zitrone, Erdbeere, u.v.a.

Die beanspruchte Flüssig-Formulierung kann als physiologisches Stärkungsmittel und in diesem Zusammenhang insbesondere in Form eines Nahrungs- oder Lebensmittels (siehe z.B. nicht abschließend EU-Richtlinie 2002/46/EG vom 10. Juni 2002) verwendet werden, ggfs. enthaltend Hilfs- und Zusatzstoffe oder eines Funktionsnahrungsmittels (Functional Food) für Mensch oder Tier.

Weitere Vorteile und Merkmale der vorliegenden Erfindung ergeben sich aufgrund der Beschreibung von Ausführungsbeispielen.

### Beispiele:

Es werden 20% (w/w) CBD in Polysorbat 80 gelöst (im Ultraschallbad für ca. 1 Std. ggf. im Wasserbad erwärmen). Anschließend werden 0,1 g der klaren CBD-Lösung mittels einer Pipette in 0,5 L Sprite® (=Limonade) gegeben. Sollte die CBD-Lösung nicht klar sein, muss noch weiter im Ultraschallbad gelöst oder ggf. im Wasserbad erwärmt werden. Es entsteht zunächst eine leichte Trübung der Trinklösung an der Oberfläche. Die Flasche wird verschlossen für ca. 45 Sek. ins Ultraschallbad gestellt, wodurch sich die komplette Flasche leicht trübt. Es setzt sich zunächst etwas weißer Schaum am Flaschenhals ab, welcher sich vor Verzehr absetzen soll. Die Flasche wird nun kurz leicht geschüttelt und ist gebrauchsfertig.
Figur 1, Elektropharmakogramm (1,0 mg/kg CBD), zeitlich relatives Veränderungsmuster:
   Zeitabhängiger Verlauf der elektrischen Leistung ("spectral power") (Ordinate) in Prozent von der 45 min. anhaltenden Vor-Medikament Basislinie (Werten) ("pre-drug baseline") in vier Hirnregionen von freibewegenden Ratten in Gegenwart eines Vehikels (4 ml/kg). Die Frequenzbereiche sind in Säulen auf der Abszisse dargestellt, nämlich delta (1), theta (2), alpha1 (3), alpha2 (4), beta1a (5), beta1b (6) und gamma spectral power (7) von links nach rechts dargestellt, je nach benannter Hirnregion. Die statistische Signifikanz ist im Vergleich zur Kontrolle (Vehikel) in Sternen dargestellt: *=p<0.05; **=p<0.01.
Figur 2, Elektropharmakogramm (5,0 mg/kg CBD), ansonsten wie Figur 1.
Figur 3, Elektropharmakogramm (15,0 mg/kg CBD), ansonsten wie Figur 1.

## Patentansprüche

1. Getränk oder dessen Grundstoff, insbesondere Energy-Drink, enthaltend eine Flüssig-Formulierung umfassend Cannabidiol und Wasser und mindestens einen Emulgator, wobei das Cannabidiol ein synthetisches Cannabidiol oder aufgereinigtes Cannabidiol aus einem Cannabis-Extrakt ist, aufweisend 50 mg/L bis 300 mg/L CBD, insbesondere 50 mg/L bis 100 mg/L CBD.

2. Getränk oder Grundstoff enthaltend eine Flüssig-Formulierung nach Anspruch 1 ausgewählt aus der Gruppe Mineralwasser, Limonade, Tonicwater, Sport-, Mineral-, Frucht-, Fruchtsaft-, Milch-, Molke, Soft- oder alkoholhaltiges Getränk, wie Bier oder als Trinkwasserzubereitung.

3. Getränk oder Grundstoff enthaltend eine Flüssig-Formulierung nach einem der Ansprüche 1 bis 2, wobei der Emulgator ausgewählt ist aus der Gruppe bestehend aus Polysorbaten, Polyoxyethylen-Sorbitanfettsäurester (Tween)- und Span-Produkten, insbesondere Tween 20, 60, 80, Gummen, Gummi arabicum, Pektinen, Stärken und modifizierten Stärken, Proteinen, Caseinaten, Bienenwachs, Lecithin und/oder Sterole.

4. Lebensmittel oder Functional Food enthaltend ein Getränk oder Grundstoff gemäß einem der Ansprüche 1 bis 3.

5. Aromamittel umfassend eine flüssig-Formulierung enthaltend Cannabidiol und Wasser und mindestens einen Emulgator, wobei das Cannabidiol ein synthetisches Cannabidiol oder aufgereinigtes Cannabidiol aus einem Cannabis-Extrakt ist, aufweisend 50 mg/L bis 300 mg/L CBD, insbesondere 50 mg/L bis 100 mg/L CBD.

## Claims

1. A beverage or its base material, particularly an energy drink, containing a liquid formulation comprising cannabidiol and water and at least one emulsifier, wherein the cannabidiol is a synthetic cannabidiol or purified cannabidiol from a cannabis extract, comprising 50 mg/L to 300 mg/L CBD, particularly 50 mg/L to 100 mg/L CBD.

2. A beverage or base material containing a liquid formulation according to claim 1, selected from the group mineral water, lemonade, tonic water, sports, mineral, fruit, fruit juice, milk, whey or soft drink or an alcoholic beverage, such as beer, or as a drinking water preparation.

3. A beverage or base material containing a liquid formulation according to one of claims 1 to 2, wherein the emulsifier is selected from the group consisting of polysorbates, polyoxyethylene sorbitan fatty acid ester (Tween) and Span products, particularly Tween 20, 60, 80, rubbers, gum arabic, pectins, starches and modified starches, proteins, caseinates, beeswax, lecithin, and/or sterols.

4. A food or functional food containing a beverage or base material according to one of claims 1 to 3.

5. A flavouring agent comprising a liquid formulation containing cannabidiol and water and at least one emulsifier, wherein the cannabidiol is a synthetic cannabidiol or purified cannabidiol from a cannabis extract, comprising 50 mg/L to 300 mg/L CBD, particularly 50 mg/L to 100 mg/L CBD.

## Revendications

1. Boisson ou sa substance de base, en particulier boisson énergisante, contenant une formulation liquide comprenant du cannabidiol et de l'eau et au moins un émulsifiant, dans laquelle le cannabidiol est un cannabidiol synthétique ou du cannabidiol purifié à partir d'un extrait de cannabis, présentant de 50 mg/L à 300 mg/L de CBD, en particulier de 50 mg/L à 100 mg/L de CBD.

2. Boisson ou substance de base contenant une formulation liquide selon la revendication 1 choisie dans le groupe comprenant eau minérale, limonade, eau tonique, boisson pour le sport, minérale, fruitée, de jus de fruits, lactée, à base de petit lait, de type soda ou alcoolisée, comme de la bière ou comme préparation d'eau potable.

3. Boisson ou substance de base contenant une formulation liquide selon l'une quelconque des revendications 1 à 2, dans laquelle l'émulsifiant est choisi dans le groupe comprenant des polysorbates, des produits de polyoxyéthylène-ester d'acide gras et de sorbitanne (Tween)-Span, en particulier Tween 20, 60, 80, des gommes, de la gomme arabique, des pectines, des amidons et amidons modifiés, des protéines, des caséinates, de la cire d'abeille, de la lécithine et/ou stérols.

4. Aliment ou aliment fonctionnel contenant une boisson ou une substance de base selon l'une des revendications 1 à 3.

5. Agent aromatisant comprenant une formulation liquide contenant du cannabidiol et de l'eau et au moins un émulsifiant, dans lequel le cannabidiol est un cannabidiol synthétique ou du cannabidiol purifié provenant d'un extrait de cannabis, comprenant de 50 mg/L à 300 mg/L de CBD, en particulier de 50 mg/L à 100 mg/L de CBD.
